# EUROPEAN PATENT SPECIFICATION

(11) **EP 3 219 809 B1**
(45) Date of publication and mention of the grant of the patent: **27.10.2021**
(21) Application number: 14901111.6
(22) Date of filing: 03.09.2014
(51) Int. Cl.: G16B 50/00, C12Q 1/68

(54) **PATHOLOGY DETERMINATION ASSISTANCE DEVICE, METHOD, PROGRAM AND STORAGE MEDIUM**
PATHOLOGIEBESTIMMUNGSHILFSVORRICHTUNG, VERFAHREN, PROGRAMM UND SPEICHERMEDIUM
DISPOSITIF, PROCÉDÉ, PROGRAMME ET SUPPORT D'ENREGISTREMENT D'AIDE À LA DÉTERMINATION DE PATHOLOGIE

(43) Date of publication of application: 20.09.2017
(73) Proprietor: Otsuka Pharmaceutical Co., Ltd., Tokyo 101-8535 (JP)
(72) Inventor: KINOSHITA, Moritoshi, Osaka-shi Osaka 540-0021 (JP); HIGASHIYAMA, Ryo, Osaka-shi Osaka 540-0021 (JP); KOGA, Daisuke, Osaka-shi Osaka 540-0021 (JP)
(74) Representative: dompatent von Kreisler Selting Werner - Partnerschaft von Patent- und Rechtsanwälten mbB
(86) International application number: PCT/JP2014/073213
(87) International publication number: WO 2016/035168

(56) References cited:
- JP-A- 2004 504 038
- JP-A- 2010 504 579
- JP-A- 2010 522 537
- US-B1- 6 303 297
- ALEXANDER M. GOUT ET AL: "PKDB: Polycystic Kidney Disease Mutation Database-a gene variant database for autosomal dominant polycystic kidney disease", HUMAN MUTATION, vol. 28, no. 7, 1 July 2007 (2007-07-01), pages 654-659, XP055468873, US ISSN: 1059-7794, DOI: 10.1002/humu.20474
- CARSTEN BERGMANN ET AL: "Algorithm for efficient PKHD1 mutation screening in autosomal recessive polycystic kidney disease (ARPKD)", HUMAN MUTATION, vol. 25, no. 3, 1 March 2005 (2005-03-01), pages 225-231, XP055468871, US ISSN: 1059-7794, DOI: 10.1002/humu.20145
- PETER C. HARRIS ET AL: "Molecular diagnostics for autosomal dominant polycystic kidney disease", NATURE REVIEWS. NEPHROLOGY, vol. 6, no. 4, 23 February 2010 (2010-02-23), pages 197-206, XP055468877, GB ISSN: 1759-5061, DOI: 10.1038/nrneph.2010.18
- YANG TAO ET AL: "Identification of novel mutations of PKD1 gene in Chinese patients with autosomal dominant polycystic kidney disease by targeted next-generation sequencing", CLINICA CHIMICA ACTA, vol. 433, 26 February 2014 (2014-02-26), pages 12-19, XP029030543, ISSN: 0009-8981, DOI: 10.1016/J.CCA.2014.02.011
- SHINSEI MINOSHIMA ET AL.: 'Shikkan Idenshi Hen'i to Shikkan Kanren Tagata no Sogo Chishiki Base no Kochiku' XP055394978 Retrieved from the Internet: <URL:http:// lifesciencedb.jp/houkoku/pdf/001/b029.pdf>
- MINOSHIMA ET AL.: 'THE KMDB/MUTATIONVIEW: A MUTATION DATABASE FOR HUMAN DISEASE GENES' NUCLEIC ACIDS RESEARCH vol. 29, no. 1, 2001, pages 327 - 328, XP055394985
- HITOSHI YOKOYAMA: 'Epidemiological and clinicopathological studies based on web registration system (J-RBR/J-KDR' JAPANESE JOURNAL OF PEDIATRIC NEPHROLOGY vol. 26, no. 2, 2013, pages 213 - 219, XP003032464
- None

## Description

### Technical Field

The present invention relates to a device for assisting determination of the pathology of a test subject, more specifically to a device for assisting determination of the pathology of a test subject by displaying a list containing information on gene mutation based on the gene sequences of the test subject, and various items of medical information in connection with the gene mutation stored in public databases.

### Background Art

In recent research with regard to gene-mutation-related diseases, studies of the relationship between diseases and gene mutations have been actively carried out, typically by analyzing the genetic information of patients. For example, with respect to polycystic kidney diseases (PKD), which are known as highly frequent hereditary kidney diseases that are also refractory diseases, PKD1 gene and PKD2 gene have been identified as genes causing autosomal dominant polycystic kidney disease (ADPKD). In ADPKD, about 85% of the gene mutation is due to abnormality of PKD1 gene, and about 15% is due to abnormality of PKD2 gene. It has been reported that the progression of the disease is accelerated by the abnormality of PKD1 gene. The Sanger sequence method (Non-patent Document 1) and the next-generation sequence analysis method (Patent Document 1 and Non-patent Document 2) have been publicly known as methods for detecting gene mutations, and mutations of PKD1 gene and PKD2 gene can be detected by these methods.

Additionally, in recent years, various public databases have disclosed study results regarding the relationship between diseases and gene mutations. For example, the Polycystic Kidney Disease (PKD) Foundation website discloses a database, regarding pathogenic mutations, that is provided by the Mayo Clinic (this database is hereinafter referred to as the "Mayo database"). Further, GenBank, which is run by the National Center for Biotechnology Information (NCBI) in the United States, discloses a database regarding various sequences. Thus, medical information regarding gene mutations can be obtained from these public databases.

### Citation List

### Patent Documents

Patent Document 1: JP2009-11230A

### Non-patent Documents

Non-patent Document 1: F. Sanger et al., "A rapid method for determining sequences in DNA by primed synthesis with DNA polymerase," Journal of Molecular Biology, April 1975, Volume 94, p. 411-446
Non-patent Document 2: "Next generation DNA sequencer - applications and the prospects for the clinical medicine," Junko Sugano-Mishima et al., Modern Media, Eiken Chemical Co., Ltd., August 2011, 57th edition, No. 8, p. 1-5

### Summary of Invention

### Technical Problem

In order to specify a pathogenic gene mutation that induces the pathology of a patient using detected gene mutation information, it is necessary to obtain various types of information, from a medical standpoint, regarding the gene mutation. Therefore, sufficient information to accurately specify a pathogenic gene mutation cannot be acquired by referring to only one public database. Further, when several to several tens of gene mutations are detected from a single patient, it is necessary to refer to a plurality of public databases and previously published academic papers for each of these several to several tens of gene mutations individually. This is significantly time-consuming; further, it is not possible to sufficiently specify a pathogenic gene mutation without databases of healthy subjects.

The present invention was made to solve the above problems, and an object thereof is to provide, for example, a device for assisting determination of pathology that enables easier specification of a pathogenic gene mutation of a test subject by displaying a list containing gene mutation information based on the gene sequences of a test subject, and various items of medical information regarding gene mutation stored in public databases; the device also refers to a database of gene mutations (polymorphism) of healthy subjects that is uniquely constructed.

### Solution to Problem

A pathology determination assistance device of the present invention for achieving the above object is a pathology determination assistance device for assisting determination of the pathology of a polycystic kidney disease as specified in claim 1. In particular, such device comprises
an extraction means configured for extracting information on gene mutation in a region related to polycystic kidney disease using sequence data showing a gene sequence of a test subject;
an acquisition means configured for acquiring, using the extracted information on gene mutation, medical information corresponding to the extracted gene mutation from a plurality of databases in which gene mutation and medical information are associated with each other; and
a list display means configured for displaying a list containing the extracted information on gene mutation and the obtained medical information,
wherein the information on gene mutation is chromosome position-based information, which includes a chromosome number, the position of mutation, and the kind of the base after mutation, and
the databases include a genetic polymorphism database of healthy subjects who are not younger than 35 years and who were confirmed by ultrasonography to be free of kidney cysts in both kidneys.

The pathology determination assistance device of the present invention preferably comprises a storage unit in which the databases are stored.

It is preferable that the information on gene mutation is chromosome position-based information, which includes a chromosome number, the position of mutation, and the kind of the base after mutation.

Further, a pathology determination assistance method of the present invention is a pathology determination assistance method for assisting determination of the pathology of a polycystic kidney disease utilizing the assistance device defined hereinbefore as specified in claim 5. In particular, such method comprises an extraction step for extracting information on gene mutation in a region related to polycystic kidney disease using sequence data showing a gene sequence of a test subject;
an acquisition step for acquiring, using the extracted information on gene mutation, medical information corresponding to the extracted gene mutation from a plurality of databases in which gene mutation and medical information are associated with each other; and
a list display step for displaying a list containing the extracted information on gene mutation and the obtained medical information, wherein the information on gene mutation is chromosome position-based information, which includes a chromosome number, the position of mutation, and the kind of the base after mutation, and the databases include a genetic polymorphism database of healthy subjects who were confirmed to be free of kidney cysts.

Further, a program of the present invention is a program for causing a computer to function as the extraction means, the acquisition means, and the list display means of the pathology determination assistance device of the present invention as defined hereinbefore.

Further, a computer-readable storage medium of the present invention is a medium in which the above program of the present invention as defined hereinbefore is stored.

### Advantageous Effects of Invention

The present invention enables display of a list of gene mutation information obtained by a test subject, as well as various items of medical information regarding the gene mutation stored in public databases, thereby exhaustively providing, as a list, various kinds of information required in determining the pathology of a test subject based on gene mutation information.

Further, as the list exhaustively showing information, not only information from existing public databases are provided, but also information from a database regarding gene mutation (polymorphism) of healthy subjects; more specifically, by performing sequence analyses of a predetermined number of healthy subjects and constructing a new unique database of healthy subjects, and referring to the thus-uniquely constructed gene mutation (polymorphism) database of healthy subjects. The present invention enables a comparison with normal gene mutations (polymorphism) observed in healthy subjects, and thereby excludes the normal gene mutations from several to several tens of detected gene mutations of a single patient, thus more easily detecting a pathogenic gene mutation in a test subject. Although patients with polycystic kidney disease have kidney cysts at birth, they often have no symptoms until they are in their 30s to 40s. Therefore, in the creation of a genetic polymorphism database of healthy subjects, it is important to select healthy subjects who are not younger than 35 years old and who were confirmed by ultrasonography to be free of kidney cysts in both kidneys.

### Brief Description of Drawings

Fig. 1 is a block diagram showing a schematic structure of a pathology determination assistance device according to an embodiment of the present invention.
Fig. 2 is a block diagram showing functions of a pathology determination assistance device according to an embodiment of the present invention.
Fig. 3 is a flow chart showing a flow of data processing performed by a pathology determination assistance device according to an embodiment of the present invention.

### Description of Embodiments

Hereinafter, the embodiments of the present invention are specifically explained with reference to the attached drawings. In the explanations and drawings below, the same reference numbers refer to the same or similar constituents, and a detailed explanation of the same or similar constituents will be omitted.

For ease of explanation, polycystic kidney disease (PKD) is used as the target disease in the pathology determination below.

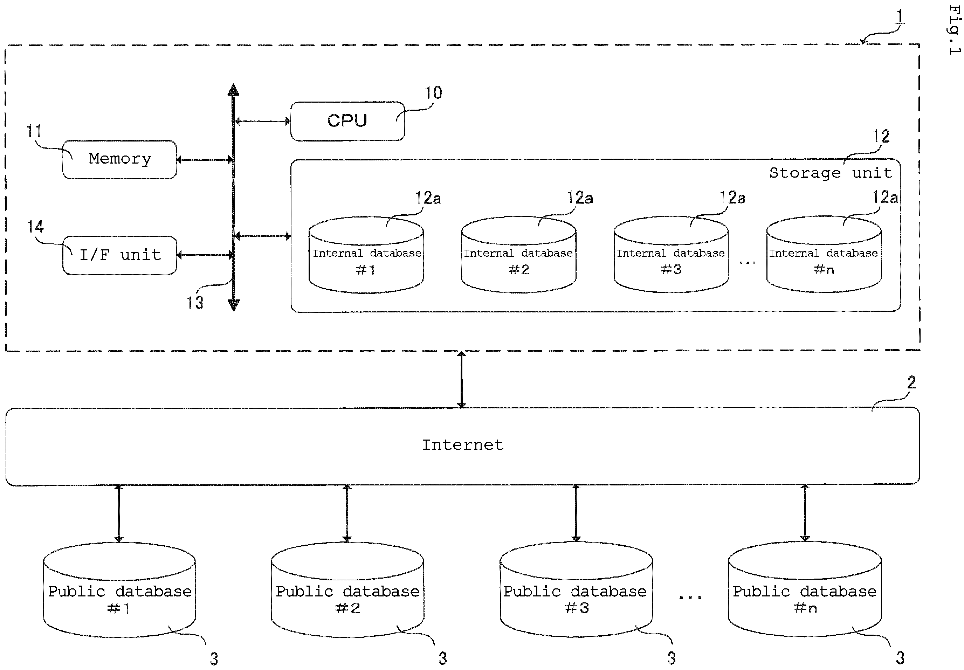

Fig. 1 is a block diagram showing a schematic structure of a pathology determination assistance device 1 according to an embodiment of the present invention. In this embodiment, the pathology determination assistance device 1 is embodied as a computer system.

The pathology determination assistance device 1 (hereinafter may simply be referred to as a "device 1") comprises a CPU 10 for performing data processing described later; a memory 11 serving as a working memory for data processing; a storage unit 12 for storing processed data; a bus 13 for transmitting data between the respective units; and an interface unit 14 (hereinafter referred to as an "I/F unit") for performing data input and output between the device 1 and external devices. Although it is not shown in Fig. 1, the pathology determination assistance device 1 also comprises various general means provided in a computer, such as an operating means (e.g., a keyboard) or a display means (e.g., a display).

In the storage unit 12, internal databases 12a are stored beforehand; in each of internal databases 12a, gene mutation information about the target disease (polycystic kidney disease (PKD)) and medical information regarding the gene mutation are associated with each other.

Further, the device 1 may also be connected to various public databases 3 via an internet 2; in this case, the internal databases 12a may store medical information regarding gene mutation that is acquired from the public databases 3, as well as gene mutation information about the target disease that is obtained by querying the public databases 3; these information items are associated with each other.

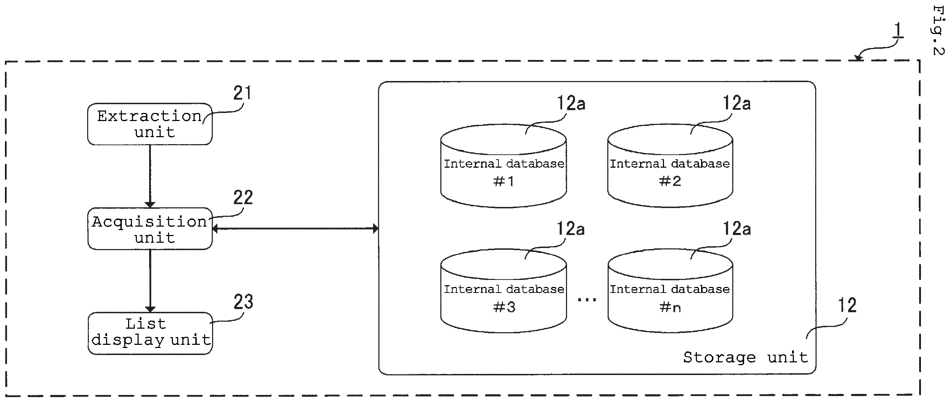

Fig. 2 is a block diagram showing functions of the device 1 according to an embodiment of the present invention. The device 1 comprises an extraction unit 21, an acquisition unit 22, and a list display unit 23. These functional blocks are embodied by installing the program of the present invention to the device 1. These functions are described later.

### 1) Gene Mutation Information

In the embodiments of the present invention, the gene mutation information is expressed based on information on chromosome position. The gene mutation information includes a chromosome number, the position (start position and end position) of the mutation in the chromosome having this number, and the type of the base after mutation. Table 1 shows an example of gene mutation information with regard to polycystic kidney disease (PKD). It is known that, in the case of PKD, PKD1 gene abnormality is present in the 16th chromosome (chr 16), and PKD2 gene abnormality is present in the fourth chromosome (chr 4).

**Table 1**

| **Contig** | **Start pos** | **End pos** | **Ref value** | **Actual value** |
|---|---|---|---|---|
| chr16 | 2143 657. | 2143 657. | G | T |
| chr16 | 2154 478. | 2154 478. | A | G |
| chr16 | 2160 494. | 2160 494. | C | T |
| chr16 | 2164 808. | 2164 808. | C | T |
| chr16 | 2166 672. | 2166 672. | G | A |
| chr16 | 2167 874. | 2167 874. | G | A |
| chr4 | 88929 305. | 88929 305. | G | A |
| chr4 | 88959 381. | 88959 381. | G | A |
| chr4 | 88979 196. | 88979 196. | C | T |
| chr4 | 88997 102. | 88997 102. | C | T |

In Table 1, the Contig column shows a chromosome number, the Start pos and End pos columns show the position of mutation (start position and end position), and the Actual value column shows the type of the base after mutation. The Ref value column shows the normal base, i.e., the type of the base before mutation, at the position.

### 2) Internal Databases

Six kinds of databases are described below as examples of various internal databases 12a that are prepared beforehand.

### Database of Healthy Japanese Subjects

Samples (e.g., blood samples) were obtained from a predetermined number (e.g., 140 subjects) of healthy Japanese subjects not younger than a predetermined age (e.g., 35 years old) having no cysts in both of their kidneys; the samples were subjected to sequence analysis by a known method, and information on the position of the detected gene mutation (for example, single nucleotide polymorphism, SNP) is converted into position information based on chromosome position information; the resulting information is stored as an internal database 12a.

When a query is given to this database, and if the gene mutation matching the query is stored in the database as a record, information as to how many subjects out of the predetermined number of subjects have the corresponding gene mutation is returned as the query result.

### Cons Paper Database

If genes of different species derived from a common ancestor were changed in the course of evolution, the proteins derived from the genes often have a common function. Such a region having a high homology between different species is called a "conserved region". The conserved region is considered important in the function of the proteins. The Cons paper (Evolutionarily conserved elements in vertebrate, insect, worm, and yeast genome. Genome ReS2005 15: 1034-1050), published by Adam Siepel et al., shows a method of expressing a state of gene region conservation using values. By quantifying the conservation states of the respective bases of the PKD1 gene region and the PKD2 gene region in the Cons paper into Cons scores, and associating the Cons scores with the position information based on the chromosome position information, the resulting information is stored as an internal database 12a.

When a query is given to this database, and if the gene mutation matching the query is stored in the database as a record, the Cons score is returned as the query result from the database. The Cons score is a real number in the range of 0 to 1, and serves as an index showing that, as the score is closer to 1, the region is more conserved, and that the presence of mutation in the base indicates a high pathogenicity.

### Mayo Database

The data of pathogenic mutations regarding PKD1 mutation and PKD2 mutation disclosed in the Mayo database is associated with position information based on chromosome position information, and is stored as an internal database 12a.

When a query is given to this database, and if the gene mutation matching the query is stored in the database as a record, a classification of determination used in the PKD foundation is returned. Examples of the classifications include "Definitely Pathogenic" and "Highly Likely Pathogenic."

### PubMed ID Database

PubMed is a database of document information created by the U.S. National Center for Biotechnology Information (NCBI). Information of pathogenic gene mutations is extracted beforehand from the hitherto-published academic papers accumulated in PubMed, and is associated with the position information based on the chromosome position information. The resulting information is stored as an internal database 12a.

When a query is given to this database, and if the gene mutation matching the query is stored in the database as a record, PubMed ID is returned. PubMed ID refers to unique ID numbers of documents accumulated in PubMed.

### Pseudogene Sequence Database

Pseudogene sequences PKD1P1, PKD1P2, PKD1P3, PKD1P4, PKD1P5, and PKD1P6, which are known pseudogene sequences with respect to PKD gene, are obtained from various public databases and are compared with PKD1 gene. The mutation sites that differ between the pseudogenes and the normal gene PKD1 are extracted, and are associated with the position information based on the chromosome position. The resulting information is stored as an internal database 12a.

When a query is given to this database, and if the gene mutation matching the query is stored in the database as a record, a result indicating that the gene mutation matching the query is a mutation derived from a pseudogene is returned as the query result from the database. When a plurality of gene mutations derived from these pseudogenes is extracted, it is likely that the pseudogenes were amplified by long-range PCR, which is described later. This serves as an index of accuracy management in gene examinations.

### GenBank Database

The information regarding the position of gene mutations of PKD1 gene and PKD2 gene obtained from GenBank database are converted to the position information based on the chromosome position information, and are stored as an internal database 12a.

When a query is given to this database, and if the gene mutation matching the query is stored in the database as a record, the rs# number is returned as a query result from the database. The rs# number refers to a reference SNP ID number, which is a universal SNP ID number defined for each SNP by the NCBI.

### 3) Performance of the pathology determination assistance device

In the explanation below, a process performed by the device 1 means a process performed by the CPU 10 of the device 1 unless otherwise specified. The CPU 10 temporarily stores necessary data (such as intermediate data being processed) in a memory 11 that serves as a working memory, and stores the data that are stored for a long period of time, such as calculation results, in the storage unit 12 as necessary. Further, in order to carry out steps S1 to S4 described below, the device 1 stores the program of the present invention in the storage unit 12 beforehand, for example, in an executable format (for example, a form in which the program can be produced by being converted from a programming language such as C language using a compiler). The device 1 carries out processing using the program stored in the storage unit 12. The program may also be installed to the device 1 from a computer-readable storage medium such as a CD-ROM; otherwise, the device 1 may be connected to the internet 2 to download the program code of the program via the internet 2.

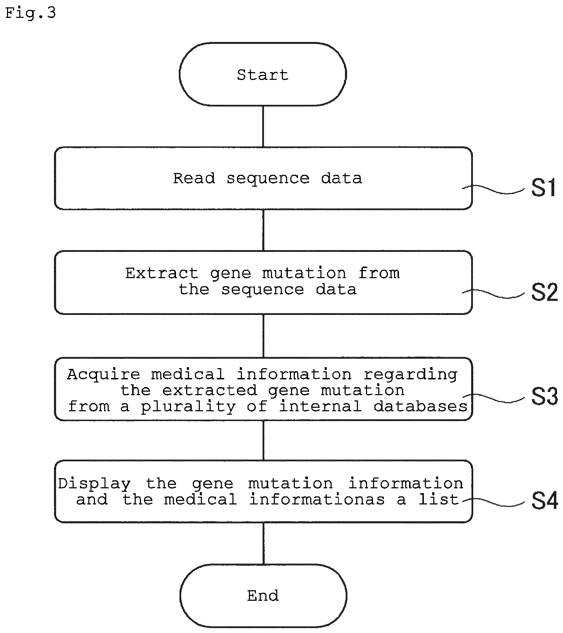

Fig. 3 is a flow chart showing a flow of data processing performed by a pathology determination assistance device according to an embodiment of the present invention. The data processing performed by the pathology determination assistance device according to the embodiment of the present invention is described in detail below based on the flow chart shown in Fig. 3.

In step S1, sequence data of a test subject is read into the device. The sequence data is created, for example, as FASTQ format data or VCF data beforehand, for example, from a sample enabling gene analysis, such as blood of the test subject, using a commercially available sequencer device, and is stored in the storage unit 12 beforehand. Alternatively, the sequence data may be acquired and read from an external device via the I/F unit 14 or the internet 2.

Creation of sequence data is explained below. In the case of polycystic kidney disease (PKD) that is used as the target disease in the determination in this embodiment, PKD1 gene and PKD2 gene have a relatively large size; therefore, a sequencer device using a next-generation sequence analysis method is more preferable, as the sequencer device for performing the detection of gene mutation, than a sequencer device using the Sanger method.

The Sanger method is a method for determining base sequence using the principle that when dideoxynucleotide is captured during the DNA replication in a sequencing reaction, the nucleic acid elongation reaction is stopped. The Sanger method ensures sufficient sensitivity for point mutation; however, the method has a problem such that if mutation other than point mutation such as deletion or insertion of the bases is present, the base sequences after the corresponding site cannot be read. Further, in the method using the Sanger method, determination of base sequence by a single kind of sequence primer is possible only for a limited chain length (up to about 500 bp). Therefore, even if only PKD1 is to be detected, it is necessary to use 90 kinds of primers for each specimen, thereby requiring a large number of processes, and thus significantly increasing the costs.

In contrast, in the analysis method called a next-generation sequence analysis method, first, exon of PKD1 gene is amplified by long-range PCR using a genomic DNA as a template, and a library of fragments of 35 bp to 400 bp is prepared. Thereafter, the base sequence is determined using a commercially available sequencer device. The next-generation sequence analysis method is capable of mass sequencing and is suitable for many kinds of analyses such as exome analysis or sequencing of genes having relatively a large size, such as PKD1 gene and PKD2 gene.

In this embodiment, the sequence data is created beforehand, for example, by a sequencer device using a next-generation sequence analysis method.

In step S2 (extraction step), the extraction unit 21 shown in Fig. 2 performs mapping and alignment of the sequence fragment length of the read sequence data (FASTQ format), thereby extracting gene mutation from the sequence data. As a specific means for extracting gene mutation, for example, known software for extracting SNP (single nucleotide polymorphism) may be used.

The extracted gene mutation information is expressed based on the information on chromosome position, which includes a chromosome number, the position (start position and end position) of the mutation in the chromosome having this number, and the type of the base after mutation. At this point in time, the extracted gene mutations include a synonymous mutation that has a mutation but has the same amino acid coded by the gene and the same protein function as those before the mutation, as well as gene mutations (polymorphism) other than the pathogenic gene mutation of polycystic kidney disease (PKD), which is the target disease in the determination.

Compared with the sequence fragment length in prior art that was about 75 bp, the sequence fragment length in this embodiment, which is set upon the extraction of gene mutation, is longer (about 400 bp) than the amplification range and amplification cross section in long-range PCR, thereby increasing the detection rate (correlation rate with respect to the ADPKD patients) from 63% to 89%.

In step S3 (acquisition step), the acquisition unit 22 shown in Fig. 2 acquires medical information regarding the gene mutation from a plurality of internal databases 12a using the extracted gene mutation information extracted in step S2. More specifically, using gene mutation information, i.e., a chromosome number, the position of mutation in the chromosome having this number, and the type of the base after mutation as search queries, the acquisition unit 22 queries each of the plurality of internal databases 12a to find any records that match the search queries. If there are any records that match the search queries in the internal databases 12a, information defined in each internal database 12a is returned as a query result.

For example, when a query regarding the presence or absence of records about mutation "T" present in position 2160494 in the 16th chromosome is given to the internal databases 12a, the gene mutation information of this query is "Contig=chr 16, Startpos=2160494, Endpos=2160494, Actual value=T." For example, in the case of the Mayo database, the device 1 determines whether the Mayo database has any records of this gene mutation information. When the records are stored in the database, the device 1 acquires a classification "Likely Neutral," which is medical information associated with the gene mutation information "Contig=chr 16, Startpos=2160494, Endpos=2160494, Actual value=T," as a query result from the internal database 12a. When there are no records in the database, the device 1 acquires information indicating that no records are stored (for example, NULL).

As in the Mayo database, for other internal databases 12a as well, the acquisition unit 22 determines whether any records of gene mutation information represented by "Contig=chr 16, Startpos=2160494, Endpos=2160494, Actual value=T" is stored in each internal database 12a. When the database has any records, the device 1 acquires medical information associated with the gene mutation. For example, in the case of the healthy Japanese subjects database, the medical information corresponds to information as to how many subjects out of the predetermined number of subjects have the gene mutation. Similarly, in the case of the Cons paper database, the medical information corresponds to the Cons score; in the case of the GenBank database, the medical information corresponds to the rs# number; and in the case of the PubMed ID database, the medical information corresponds to the PubMed ID.

In step S4 (list display step), the list display unit 24 shown in Fig. 2 displays a list containing gene mutation information extracted in step S2 and medical information obtained in step S3. Table 2 shows an example of items in the list.

**Table 2**

| **Contig** | **Start pos** | **End pos** | **Ref value** | **Actual value** | **Actual** | | | | |
|---|---|---|---|---|---|---|---|---|---|
| | | | | | **DB #1** | **DB #2** | **DB #3** | **DB #4** | **DB #5** |
| | | | | | **Mayo Classification** | **Id** | **Jap Ref** | **Cons** | **PMID** |
| chr16 | 2143 657 | 2143 657 | G | T | | | | 0 | |
| chr16 | 2154 478 | 2154 478 | A | G | Likely Neutral | rs4786209 | 100/140 | 0 | |
| chr16 | 2160 494 | 2160 494 | C | T | Likely Neutral | rs79884128 | 54/140 | 0.023622 | 22185115 |
| chr16 | 2164 808. | 2164 808. | C | T | | rs40433 | 24/140 | 0 | |
| chr16 | 2166 672 | 2166 672 | G | A | Likely Neutral | rs4787158 | 15/140 | 0 | |
| chr16 | 2167 874 | 2167 874 | G | A | Likely Neutral | | | 0 | |
| chr4 | 88929 305 | 88929 305. | G | A | Likely Neutral | rs2728118 | 90/140 | 0.267717 | 22008521 |
| chr4 | 88959 381 | 88959 381 | G | A | | rs2725221 | 122/140 | 0 | 22008521 |
| chr4 | 88979 196 | 88979 196 | C | T | Definitely Pathogenic | rs146396414 | | 1 - Likely pathogenic | |
| chr4 | 88997 102 | 88997 102 | C | T | | rs2728121 | 101/140 | 0 | |

In Table 2, the Contig column shows a chromosome number, the Start pos and End pos columns show the position (start position and end position) of mutation, and the Actual value column shows the type of the base after mutation. The Ref value column shows the normal base, i.e., the type of the base before mutation, at the position. The "Actual" and "DB #1" to "DB #5" columns show medical information obtained from the internal databases 12a. These columns show, from left to right, classification according to the Mayo database, the rs# number according to the GenBank database, the number of gene mutation carriers according to the healthy Japanese subjects database, the Cons score according to the Cons paper database, and the PubMed ID according to the PubMed ID database.

For example, referring to Table 2 regarding mutation "T" in position 2160494 in the 16th chromosome, the row specified by "Contig=chr 16, Startpos=2160494, Endpos=2160494, Actual value=T" shows, as a list, classification (Likely Neutral) according to the Mayo database, the rs# number (rs 79884128), the number of gene mutation carriers among Japanese (54/140), the Cons score (0.023622), and the PubMed ID (22185115).

Further, Table 2 shows one to several tens of gene mutations extracted from the sequence data of the test subject. Each of the extracted gene mutations is displayed while being individually associated with medical information acquired from the internal databases 12a. The information items exhaustively listed in Table 2 are various kinds of information, from a medical standpoint, required to determine the pathology of the patient.

As described above, the present invention enables display of a list containing gene mutation information obtained from a test subject and various items of medical information regarding the gene mutation stored in public databases or the like, thereby exhaustively providing, in the form of a list, various items of information required to determine the pathology of the test subject based on gene mutation information. Therefore, with the present invention, it becomes unnecessary to individually refer to a plurality of public databases or academic papers for the individual gene mutations, thereby reducing the labor required for the pathology determination of the test subject.

Further, since these information items required for the determination are exhaustively displayed, it becomes unnecessary to stop the determination work in each step of referring to a plurality of public databases or academic papers, thereby allowing the user to focus more on the determination work.

Although a list of gene mutation information extracted in step S2 and medical information obtained in step S3 are displayed in step S4 in the embodiment described above, the items of gene mutation information in the list may be different from those in this embodiment. In addition to these information items, any items required for the determination may be suitably selected from various items, such as effects of mutation (Effect), discrimination between PKD1 gene and PKD2 gene (Region), codon mutation (Codon), amino acid mutation (Aa), nucleotide mutation (Nuc ch), and protein change (Prot ch), to be added to the list. Table 3 shows an example of a list including these additional items.

| **Contig** | **Start pos** | **End pos** | **Ref value** | **Actual value** | **Actual** | | | | | | | | | | |
|---|---|---|---|---|---|---|---|---|---|---|---|---|---|---|---|
| | | | | | | | | | | | **DB #1** | **DB** #**2** | **DB #3** | **DB #4** | **DB #5** |
| | | | | | **Effect** | **Region** | **Codon** | **Aa** | **Nuc ch** | **Prot ch** | **Mayo Classification** | **Id** | **Jap Ref** | **Cons** | **PMID** |
| chr16 | 2143 657. | 2143 657. | G | T | Non syn cod | PKD1 | gCt/gAt | A3635D | | | | | | 0 | |
| chr16 | 2154 478 | 2154 478 . | A | G | Intron | PKD1 | | | 8161+21T>C | Likely Silent | Likely Neutral | rs4786209 | 100/140 | 0 | |
| chr16 | 2160 494 | 2160 494. | C | T | Syn coding | PKD1 | acG/acA | T1558T | 4674G>A | Thr1558Thr | Likely Neutral | rs79884128 | 54/140 | 0.023622 | 22185115 |
| chr16 | 2164 808. | 2164 808. | C | T | Non syn cod | PKD1 | cGg/cAg | R739Q | | | | rs40433 | 24/140 | 0 | |
| chr16 | 2166 672. | 2166 672. | G | A | Intron | PKD1 | | | 1607-27C>T | Likely Silent | Likely Neutral | rs4787158 | 15/140 | 0 | |
| chr16 | 2167 874 . | 2167 874 . | G | A | Syn coding | PKD1 | ctC/ctT | L373L | 1119C>T | Leu373Leu | Likely Neutral | | | 0 | |
| chr4 | 88929 305. | 88929 305. | G | A | Syn coding | PKD2 | ggG/ggA | G140G | 420G>A | Gly140Gly | Likely Neutral | rs2728118 | 90/140 | 0.267717 | 22008521 |
| chr4 | 88959 381. | 88959 381 | G | A | Intron | PKD2 | | | | | | rs2725221 | 122/140 | 0 | 22008521 |
| chr4 | 88979 196. | 88979 196. | C | T | Stop gained | PKD2 | Cga/Tga | R654* | 1960C>T | Arg654X | Definitely Pathogenic | rs146396414 | | 1 - Likely pathogenic | |
| chr4 | 88997 102. | 88997 102. | C | T | Utr3 prime | PKD2 | | | | | | rs2728121 | 101/140 | 0 | |

Further, although the data processing shown in Fig. 3 is performed by the CPU 10 in the embodiment described above, the data processing may also be performed in such a manner that the processing performed by the CPU 10 is first divided to separate functions, a dedicated electronic circuit is created for each function, and these electronic circuits execute the divided steps of the data processing in Fig. 3.

Further, although a stand-alone system in which the internal databases 12a are stored in the storage unit 12 of the device 1 is used in the embodiment described above, the storage for storing the internal databases 12a is not limited to the storage unit 12. For example, a network-type system may be used in which the internal databases 12a are stored in another computer device separated from the device 1, and obtained by accessing the other computer device through the internet 3.

Further, in the embodiment described above, the information on gene mutation with respect to the target disease and the medical information in connection with the gene mutation are associated with each other and stored in the internal databases 12a beforehand; however, it is not necessary to fix the information items in the internal database 12a; instead, the information items may be dynamically and regularly updated, for example, through the internet 3. Examples of the means for dynamically updating the contents of the internal databases 12a includes creation of an automation program in which update procedures are written in a script language. In this case, the automation program is stored in the storage unit 12 in the device 1, and is regularly booted to automatically access the public databases 3 so as to automatically collect information required for the update of the internal databases 12a from the public databases 3, thereby updating the contents of the internal databases 12a.

Further, although the operating means and the display means are described as separate structures in the embodiment described above, the operating means and the display means may be unified to form a touch-panel-type structure.

Further, in the embodiment described above, text information is displayed in step S4 as the information items to be displayed as a list; however, it may also be configured such that predetermined processing associated with the text information is suitably executed. For example, it may be configured such that, when a list of PubMed ID is displayed, by specifying a PubMed ID number using, for example, an operating means (mouse), the data files of academic papers associated with the ID number are displayed.

### Description of Reference Numerals

- 1.: Pathology determination assistance device
- 2.: Internet
- 3.: Public database
- 10.: CPU
- 11.: Memory
- 12.: Storage unit
- 12a.: Internal database
- 13.: Bus
- 14.: Interface unit
- 21.: Extraction unit (extraction means)
- 22.: Acquisition unit (acquisition means)
- 23.: List display unit (list display means)

## Claims

1. A pathology determination assistance device for assisting determination of pathology of polycystic kidney disease, comprising:
an extraction means configured for extracting information on gene mutation in a region related to polycystic kidney disease using sequence data showing a gene sequence of a test subject;
an acquisition means configured for acquiring, using the extracted information on gene mutation, medical information corresponding to the extracted gene mutation from a plurality of databases in which gene mutation and medical information are associated with each other; and
a list display means configured for displaying a list containing the extracted information on gene mutation and the obtained medical information,
wherein the information on gene mutation is chromosome position-based information, which includes a chromosome number, the position of mutation, and the kind of the base after mutation, and
the databases include a genetic polymorphism database of healthy subjects who are not younger than 35 years and who were confirmed by ultrasonography to be free of kidney cysts in both kidneys.

2. The pathology determination assistance device according to claim 1, wherein the pathology determination assistance device comprises a storage unit in which the databases are stored.

3. The pathology determination assistance device according to claim 1 or 2, wherein the information being displayed in the list includes an information as to how many subjects out of a predetermined number of the subjects who were confirmed to be free of kidney cysts have the gene mutation which corresponds to the extracted gene mutation.

4. The pathology determination assistance device according to any one of claims 1 to 3, the databases further include a pseudogene sequence database.

5. A pathology determination assistance method for assisting determination of pathology of polycystic kidney disease utilizing the assistance device of any one of claims 1 to 4, comprising:
an extraction step for extracting information on gene mutation in a region related to polycystic kidney disease using sequence data showing gene sequences of a test subject;
an acquisition step for acquiring, using the extracted information on gene mutation, medical information corresponding to the extracted gene mutation from a plurality of databases in which gene mutation and medical information are associated with each other; and
a list display step for displaying a list containing the extracted information on gene mutation and the obtained medical information,
wherein the information on gene mutation is chromosome position-based information, which includes a chromosome number, the position of mutation, and the kind of the base after mutation, and
the databases include a genetic polymorphism database of healthy subjects who were confirmed to be free of kidney cysts.

6. The pathology determination assistance method according to claim 5, wherein the information being displayed in the list includes an information as to how many subjects out of a predetermined number of the subjects who were confirmed to be free of kidney cysts have the gene mutation which corresponds to the extracted gene mutation.

7. The pathology determination assistance method according to claim 5 or 6, the databases further include a pseudogene sequence database.

8. A program for causing a computer to function the extraction means, the acquisition means, and the list display means of the pathology determination assistance device according to any one of claims 1 to 4.

9. A computer-readable storage medium in which the program according to claim 8 is stored.

## Patentansprüche

1. Hilfsvorrichtung zur Pathologiebestimmung zur Unterstützung bei der Pathologiebestimmung von polyzistischer Nierenerkrankung, umfassend:
ein Extraktionsmittel, das konfiguriert ist, unter Verwendung von Sequenzdaten, die eine Gensequenz eines Versuchssubjekts zeigen, Informationen über Genmutation in einem Bereich zu extrahieren, der sich auf polyzistische Nierenerkrankung bezieht;
ein Erfassungsmittel, das konfiguriert ist, unter Verwendung der extrahierten Informationen über Genmutation medizinische Informationen, die der extrahierten Genmutation entsprechen, aus einer Vielzahl von Datenbanken zu erfassen, bei denen Genmutation und medizinische Informationen zueinander in Bezug gesetzt werden; und
ein Listenanzeigemittel, das konfiguriert ist, eine Liste anzuzeigen, die die extrahierten Informationen über Genmutation und die erhaltenen medizinischen Informationen anzeigt,
wobei die Informationen über Genmutation auf Chromosomenpositionen basierte Informationen sind, die eine Chromosomenzahl, die Position der Mutation und die Art der Basis nach einer Mutation einschließen, und
die Datenbanken eine Datenbank zu genetischem Polymorphismus gesunder Subjekte einschließen, die nicht jünger als 35 Jahre sind und bei denen durch Ultrasonographie bestätigt wurde, dass diese in beiden Nieren frei von Nierenzysten sind.

2. Hilfsvorrichtung zur Pathologiebestimmung nach Anspruch 1, wobei die Hilfsvorrichtung zur Pathologiebestimmung eine Speichereinheit umfasst, in der die Datenbanken gespeichert sind.

3. Hilfsvorrichtung zur Pathologiebestimmung nach Anspruch 1 oder 2, wobei die in der Liste angezeigten Informationen eine Information darüber einschließen, wie viele Subjekte aus einer vorbestimmten Anzahl von Subjekten, bei denen bestätigt wurde, dass diese frei von Nierenzysten sind, die Genmutation aufweisen, die der extrahierten Genmutation entspricht.

4. Hilfsvorrichtung zur Pathologiebestimmung nach einem der Ansprüche 1 bis 3, wobei die Datenbanken weiter eine Pseudogensequenzdatenbank einschließen.

5. Hilfsverfahren zur Pathologiebestimmung zur Unterstützung bei der Pathologiebestimmung von polyzistischer Nierenerkrankung unter Verwendung der Hilfsvorrichtung nach einem der Ansprüche 1 bis 4, umfassend:
einen Extraktionsschritt zum Extrahieren von Informationen über Genmutation in einem Bereich, der sich auf polyzistische Nierenerkrankung bezieht, unter Verwendung von Sequenzdaten, die Gensequenzen eines Versuchssubjekts zeigen;
einen Erfassungsschritt zum Erfassen, unter Verwendung der extrahierten Informationen über Genmutation, von medizinischen Informationen, die der extrahierten Genmutation entsprechen, aus einer Vielzahl von Datenbanken, bei denen Genmutation und medizinische Informationen zueinander in Bezug gesetzt werden; und
einen Listenanzeigeschritt zum Anzeigen einer Liste, die die extrahierten Informationen über Genmutation und die erhaltenen medizinischen Informationen anzeigt,
wobei die Informationen über Genmutation auf Chromosomenpositionen basierte Informationen sind, die eine Chromosomenzahl, die Position der Mutation und die Art der Basis nach einer Mutation einschließen, und
die Datenbanken eine Datenbank zu genetischem Polymorphismus gesunder Subjekte einschließen, bei denen bestätigt wurde, dass diese frei von Nierenzysten sind.

6. Hilfsverfahren zur Pathologiebestimmung nach Anspruch 5, wobei die in der Liste angezeigten Informationen eine Information darüber einschließen, wie viele Subjekte aus einer vorbestimmten Anzahl von Subjekten, bei denen bestätigt wurde, dass diese frei von Nierenzysten sind, die Genmutation aufweisen, die der extrahierten Genmutation entspricht.

7. Hilfsverfahren zur Pathologiebestimmung nach Anspruch 5 oder 6, wobei die Datenbanken weiter eine Pseudogensequenzdatenbank einschließen.

8. Programm, um einen Computer zu veranlassen, das Extraktionsmittel, das Erfassungsmittel und das Listenanzeigemittel der Hilfsvorrichtung zur Pathologiebestimmung nach einem der Ansprüche 1 bis 4 zu betreiben.

9. Computerlesbares Speichermedium, in dem das Programm nach Anspruch 8 gespeichert ist.

## Revendications

1. Dispositif d'aide à la détermination de pathologie pour aider à la détermination d'une pathologie d'une maladie polykystique des reins, comprenant :
un moyen d'extraction configuré pour extraire des informations sur une mutation génique dans une région liée à une maladie polykystique des reins en utilisant des données de séquence montrant une séquence de gènes d'un sujet de test ;
un moyen d'acquisition configuré pour acquérir, en utilisant les informations extraites sur la mutation génique, des informations médicales correspondant à la mutation génique extraite à partir d'une pluralité de bases de données dans lesquelles la mutation génique et les informations médicales sont associées les unes aux autres ; et
un moyen d'affichage de liste configuré pour afficher une liste contenant les informations extraites sur la mutation génique et les informations médicales obtenues,
dans lequel les informations sur la mutation génique sont des informations basées sur la position des chromosomes, qui incluent un numéro de chromosome, la position de la mutation et le type de base après mutation, et
les bases de données incluent une base de données de polymorphismes génétiques de sujets sains qui n'ont pas moins de 35 ans et qui ont été confirmés par échographie comme étant exempts de kystes rénaux dans les deux reins.

2. Dispositif d'aide à la détermination de pathologie selon la revendication 1, dans lequel le dispositif d'aide à la détermination de pathologie comprend une unité de stockage dans laquelle les bases de données sont stockées.

3. Dispositif d'aide à la détermination de pathologie selon la revendication 1 ou 2, dans lequel les informations étant affichées dans la liste incluent une information sur le nombre de sujets sur un nombre prédéterminé de sujets qui ont été confirmés comme étant exempts de kystes rénaux qui présentent la mutation génique qui correspond à la mutation génique extraite.

4. Dispositif d'aide à la détermination de pathologie selon l'une quelconque des revendications 1 à 3, dans lequel les bases de données incluent en outre une base de données de séquences de pseudogènes.

5. Procédé d'aide à la détermination de pathologie pour aider à la détermination d'une pathologie d'une maladie polykystique des reins utilisant le dispositif d'aide selon l'une quelconque des revendications 1 à 4, comprenant :
une étape d'extraction pour extraire des informations sur une mutation génique dans une région liée à une maladie polykystique des reins en utilisant des données de séquence montrant des séquences de gènes d'un sujet de test ;
une étape d'acquisition pour acquérir, en utilisant les informations extraites sur la mutation génique, des informations médicales correspondant à la mutation génique extraite à partir d'une pluralité de bases de données dans lesquelles la mutation génique et les informations médicales sont associées les unes aux autres ; et
une étape d'affichage de liste pour afficher une liste contenant les informations extraites sur la mutation génique et les informations médicales obtenues,
dans lequel les informations sur la mutation génique sont des informations basées sur la position des chromosomes, qui incluent un numéro de chromosome, la position de la mutation et le type de la base après mutation, et
les bases de données incluent une base de données de polymorphismes génétiques de sujets sains qui ont été confirmés comme étant exempts de kystes rénaux.

6. Procédé d'aide à la détermination de pathologie selon la revendication 5, dans lequel les informations étant affichées dans la liste incluent une information sur le nombre de sujets sur un nombre prédéterminé de sujets qui ont été confirmés comme étant exempts de kystes rénaux qui présentent la mutation génique qui correspond à la mutation génique extraite.

7. Procédé d'aide à la détermination de pathologie selon la revendication 5 ou 6, dans lequel les bases de données incluent en outre une base de données de séquences de pseudogènes.

8. Programme pour amener un ordinateur à exploiter le moyen d'extraction, le moyen d'acquisition et le moyen d'affichage de liste du dispositif d'aide à la détermination de pathologie selon l'une quelconque des revendications 1 à 4.

9. Support de stockage lisible par ordinateur dans lequel le programme selon la revendication 8 est stocké.
